# EUROPEAN PATENT APPLICATION

(11) **EP 1 207 205 A1**
(43) Date of publication of application: **22.05.2002**
(21) Application number: 00204097.0
(22) Date of filing: 20.11.2000
(51) Int. Cl.: C12N 15/86

(54) **Adenoviral replicons**

(71) Applicant: Crucell Holland B.V., 2333 CN Leiden (NL)
(72) Inventor: Havenga, Menzo Jans Emco, 2401 KG Alphen a/d Rijn (NL); Brus, Ronald Hendrik Peter, 2252 EB Voorschoten (NL)
(74) Representative: Prins, Adrianus Willem

(57) **Abstract**

The invention provides a method for identifying an adenoviral replicon capable of eliminating a target cell, comprising contacting a representative cell with said adenoviral replicon and observing any detrimental effect. Once said replicon has been identified, it can be used to specifically eliminate certain cells involved in disease, for instance tumor cells. Preferably, said replicon contacts, enters and replicates predominantly in diseased cells, causing a detrimental effect in said cells, while in non-diseased cells no or a tolerable detrimental effect is induced. Preferably, said adenoviral replicon comprises a recombinant adenovirus with a fusion between DNA from Ad5 and subgroup B adenoviral DNA. Methods for producing and purifying a replicon according to the invention is also herewith provided.

## Description

The invention relates to the field of molecular genetics and medicine. In particular, the present invention relates to the field of gene therapy, more in particular to gene therapy using viral vectors, especially adenoviruses to treat cancer.

On a yearly basis millions of people world wide suffer from cancer. Although the incidence of a particular form of cancer can vary regionally, breast-, lung-, colon-, prostate, pancreas-, and bladder cancer are among the cancer types having the highest incidence. With an overall average survival chance of 50%, cancer claims the second highest mortality rate in the world. As the incidence increases with age, its incidence will further increase as the result of an aging population. Treatment for cancer is generally conventional i.e. surgery, radiotherapy, chemotherapy or combinations hereof.

The formation of a tumor is a highly complex process that presumably is initiated through a number of mutations in the DNA of a single cell. The transformation of this single cell is accompanied by the fact that the cell also becomes immortalized resulting in that the cell no longer responds to signals from outside or from neighbouring cells to stop proliferating. Moreover, the cell has lost the ability that is normally present in non-diseased cells to go into programmed cell death or apoptosis when mutations in the DNA occur that can no longer be handled by DNA repair mechanisms.

During growth of the tumor it becomes vascularized by formation of blood vessels that are sprouting in the tumor, mainly driven by the cytokine production of the tumor cells. The blood supply provides the tumor with nutrients and oxygen and also allows invasion of tumor cells to other tissues (metastasis).

A relatively novel way to treat cancer is by means of gene therapy. Many gene therapy based strategies to specifically target tumor cells in humans involve the use of DNA delivery vehicles like viruses. One of the major focuses in this field of anti-cancer gene therapies has been on recombinant adenoviruses, since these viruses are well known for their ability to transfer DNA into a cell in a very efficient manner. Recombinant adenoviruses can be produced in large quantities and to very high titers in so-called packaging cells. Moreover, the genome of the adenovirus leaves enough room to introduce therapeutic genes. Anti-cancer therapies that have been developed to date that involve recombinant adenoviruses are mainly based on adenovirus serotype 2 or serotype 5, since their genomes are well characterized and their life cycle and cell binding properties are well understood.

To date six different subgroups of human adenoviruses have been proposed which in total encompasses 51 different serotypes (De Jong et al, 1999). A serotype is defined on the basis of its immunological distinctiveness as determined by quantitative neutralization with animal derived antisera (horse, rabbit etc). If neutralization shows a certain degree of cross-reaction between two viruses, distinctiveness of serotype is assumed if the hemagglutinins are unrelated as shown by the lack of cross-reaction of hemagglutination inhibition or when substantial biophysical/ biochemistry differences in DNA exists (Francki et al 1991). The nine serotypes identified last (serotypes 42 to 51) were isolated from HIV infected patients (Hierholzer et al 1988; Schnurr et al 1993; De Jong et al 1999). For reasons not well understood, most of such immune-compromised patients shed adenoviruses that were rarely or never isolated for immune-competent individuals (Hierholzer et al 1988 and 1992; Khoo et al 1995; De Jong et al 1998). It should be noted that the Ad51 that is described here is Ad50 in the publication of De Jong et al (1999). Likewise, Ad50 that is described here is Ad51 in the publication of De Jong et al (1999).

The recombinant adenoviruses that are used for gene therapy purposes generally do not replicate in human cells, since these adenoviruses lack genes such as the genes in the E1 region that are required for replication. The replication supporting genes are provided only by the packaging cells. These non-replicating recombinant adenoviruses usually contain therapeutic genes in and instead of the E1 region. Due to this, the virus stock is produced in the packaging cell line and subsequently used to infect cells that do not support replication and packaging but instead express the therapeutic genes encoded by the heterologous DNA that is been introduced in the recombinant adenovirus.

Although the adenoviruses seem to be promising tools in gene therapy for the treatment of tumors, an increasing number of disadvantages have become apparent over the last few years. Most adenoviruses that are injected directly into the bloodstream end up in liver cells instead of the tumor cells where their effects are needed. Apparently, the different adenovirus serotypes can differ significantly in their cell type specificity. Furthermore, therapies in which it is necessary to repeat the treatment with a number of doses of the same adenovirus render the immune system of the treated individual to very efficiently neutralize adenoviruses that are injected in following injections.

Another drawback of the use of adenoviruses is that the recombinant adenoviruses used to date only target the outside of the tumor. The cells on the inside of the tumor remain non-infected due to penetration limitations of the recombinant adenovirus. Although with these strategies tumor regression has been observed in many animal models it has not been demonstrated that complete tumor eradication could be obtained in *in vivo* animal models with a significant tumor mass. The cells on the inside will grow out eventually unless treated differently.

These features result in very low efficiencies of this kind of gene therapy strategies and result furthermore in the necessity of very high doses of viruses that are applied to finally obtain some sort of significant therapeutic effect (D'Ambrosio et al 1982).

The present invention provides means and methods for the identification, the production, the purification and the use of an adenoviral replicon capable of eliminating a target cell. In one aspect the invention provides a method for identifying an adenoviral replicon capable of eliminating a target cell, comprising contacting a representative cell with said adenovirus replicon and observing any detrimental effect. An adenoviral replicon comprises an adenoviral nucleic acid having all necessary elements for replication of said nucleic acid in a cell. Elements for replication of said nucleic acid may partly be present in trans. In a preferred embodiment said adenoviral replicon comprises an element allowing packaging of said adenoviral replicon into a virus-like particle. Non-limiting examples of an adenoviral replicon are wild type adenoviruses. In another preferred embodiment said adenoviral replicon comprises an element allowing said adenoviral replicon and/or said virus-like particle to contact and to enter a cell. In a more preferred embodiment said adenoviral replicon and/or said virus-like particle preferably contacts and enters a tumor cell. In an even more preferred embodiment said adenoviral replicon and/or said virus-like particle predominantly replicates in tumor cells. A representative cell is defined as a cell that indicates which adenoviral replicon has, or has a more pronounced, detrimental effect on a certain kind of aberrant cell. Preferably said representative cell comprises a human cell. Human cells are better predictors for human disease than non-human cells. Preferably, said representative cell comprises a tumor cell. Adenoviral replicons capable of conferring detrimental effects on said representative cell can be used to treat tumor types. Said tumor types comprise at least those tumor types that said representative cell is a derivative or analogue of. Non-limiting examples of representative cells are A549 (Human lung epithelia, ATCC CCL-185), Capan-1 (Human pancreas, ATCC HTB-79), MCF-7 (Human breast, ATCC, HTB-22), BxPC3 (Human pancreas, ATCC, CRL-1687), U87 (Human astroglioma ECACC no.89081402), SK-N-MC (Human neuroblastoma, ATCC HTB-110), MIA-PaCa-2 (Human pancreas, ATCC CRL-1420), Hs766T (Human pancreas, ATCC HTB-134), HeLa (Human Cervix, ATCC, CCL-2), HepG2 (Human liver ECACC no.85011430), SK-Cha-1 and Mz-Cha-1 (Both human cholangio carcinoma), OE19 and OE33 (Both human oesophagus ECACC no.96071721 and 96070808 resp.) and TE-1, TE-2 and T-tn (All three human squamous cell carcinoma).

In one aspect the present invention provides wild type or recombinant adenoviral replicons such as adenoviruses capable of specifically causing a detrimental effect in diseased cells such as tumor cells, while in non-diseased cells no or a tolerable detrimental effect is induced. In a preferred embodiment said specificity is provided by a specific recognition of target cells, a specific replication in target cells and/or a specific infection of said target cell. The invention provides the use of said adenoviral replicons in the production of a pharmaceutical composition and the use of said pharmaceutical composition for gene therapy purposes. According to the invention said adenoviral replicons can be of wild type origin or are made recombinant and/or chimaeric by modifications in the DNA. Examples of modifications in the DNA of said adenoviral replicons or said adenoviruses are modifications in the genes encoding capsid proteins or in genes encoding proteins involved in replication.

In another aspect the invention provides a method for producing an adenoviral replicon of the invention comprising contacting a producer cell with said adenoviral replicon and obtaining produced adenoviral replicon from said producer cell and/or culture medium. Said producer cell enables the generation of very high titers of said adenoviral replicon in said producer cell. Upon lysis of said producer cell produced adenoviral replicons become present in said culture medium, from which said produced adenoviral replicons are purified. In a preferred aspect the invention provides a method for producing an adenoviral replicon of the invention comprising contacting a producer cell, wherein said producer cell comprises a representative cell or derivative thereof. A representative cell that generates significant high titers of said adenoviral replicon for the generation of pharmaceutical compositions for the use in the treatment of aberrant cells is preferably used as a producer cell for said adenoviral replicon. In a more preferred aspect the invention provides a method for producing an adenoviral replicon of the invention comprising contacting a producer cell, wherein said producer cell comprises a functional E1 region derived from adenovirus. Preferably said producer cell comprises PER.C6 or a derivative thereof. In another aspect the invention provides a method for producing an adenoviral replicon of the invention comprising contacting a producer cell, wherein said producer cell comprises A549 or U87 or a derivative thereof.

The invention provides the use of a producer cell such as PER.C6, A549 and U87 cell or another representative cell that supports the replication and packaging of said wild type, recombinant and/or chimaeric adenoviral replicons and adenoviruses. The generation of said adenoviral replicons in said producing cells results in significant titers of adenoviral replicons that can be used in pharmaceutical compositions that can be used for the treatment of aberrant or neoplastic cells such as tumor cells.

Adenoviral replicons provided by the invention can replicate in tumor cells and in representative cells resulting in a detrimental effect such as apoptosis and/or lysis of said tumor cells. Importantly, a detrimental effect can also be the induction of necrosis, cell cycle arrest, DNA fragmentation, apoptosis or lysis in tumor cells present in said solid tumors that were not infected during the first administration. This results in an eradication and removal of a larger tumor mass than could be obtained by using non-replicating recombinant adenoviruses that only target cells on the outside of the tumor. Lysis of cells that were not transduced during the first administration is due to the infection, replication and packaging of the viruses that were produced in the cells that were infected at the first administration. This results in a deeper penetration and a therapeutic effect that is deeper into the tumor than was obtained thus far with recombinant non-replicating adenoviruses. The present invention further provides a diminished replication ability in non-tumor cells of the wild type or recombinant adenoviruses provided by the invention.

The recombinant adenoviruses provided may carry mutations in the E1 and/or E2 and/or E3 regions of the adenoviral genome. The mutations in the E1 region preferably are in the E1A and/or E1B gene. In one embodiment the invention provides the use of an adenoviral replicon preferably a recombinant adenovirus in which the E1 region of the adenovirus is modified in such a way that said adenoviral replicon or adenovirus replicates more efficiently in tumor cells than in non-tumor cells. The modifications provided by the present invention involve alterations like deletions, pointmutations and/or additions. In an even more preferred embodiment the present invention provides the use of a mutation in the E1B 55K encoding gene that enables the specific replication in tumor cells that lack functional tumor suppressor proteins like p53.

The invention also provides the use of recombinant chimaeric adenoviral replicons or adenoviruses of the invention in which the capsid derived from adenovirus has been modified to specifically target a diseased cell such as a tumor cell. The modification of the capsid may involve the exchange of the genes encoding fiber and/or hexon and/or penton. In one embodiment of the present invention an recombinant adenoviral replicon or adenovirus is built up from parts from different serotype subgroups. In a more preferred embodiment of the invention a gene encoding the fiber protein of adenovirus serotype 5 (subgroup C) is (partly) replaced by a (part of) a gene encoding the fiber protein from another serotype. In an even more preferred embodiment (part of) a gene encoding the fiber protein of adenovirus serotype 5 is replaced by (part of) a gene encoding the fiber protein from an adenoviral serotype from subgroup B such as Ad11, Ad16, Ad35 and Ad51.

The present invention further provides adenoviral replicons or adenoviruses harboring mutations in regions that are involved in replication such as the Inverted Terminal Repeats (ITR's) that are present on both ends of the viral genome and the genes encoding E2 proteins, the precursor terminal protein pTP and the DNA polymerase pol protein. These mutations result in a more specific replication in aberrant cells such as tumor cells as compared to non-aberrant cells.

In yet another embodiment of the present invention the adenoviral genome is modified by mutations in regions harboring genes that are necessary for the adenovirus to replicate and escape a defense mechanism of the transduced cell and to escape at least part of the immune system of the treated individual. In a more preferred embodiment the invention provides an adenovirus with a mutation in the E1 region and/or the E2 and/or the E3 region besides point mutations and/or deletions in other regions of the adenoviral genome to enhance replication specificity in tumor cells.

The invention further provides the selection and use of recombinant adenoviral replicons or adenoviruses that have incorporated heterologous DNA in regions in the genome, wherein said regions are not involved in replication processes. In one embodiment of the present invention the heterologous DNA is incorporated in the E3 region. Because E3 is involved in immune suppression, the replicating virus expresses proteins with by-stander effects and adds to the enhancement of immune responses from the treated individual towards the infected tumor cell. In another embodiment heterologous DNA that is incorporated in the adenoviral genome such as the E3 region encodes for antigens such as gp100, CAMEL, PRAME, Mart-1, Mage-1, Melan-A, tyrosinase (Kawakami 2000, Tsukamoto et al 2000, Jager et al 2000, Mackensen et al 2000, Yang et al 2000, Tuting et al 1999, Aarnoudse et al 1999). In another preferred embodiment heterologous DNA encodes an antigen that will stimulate the immune system of the treated individual to target the infected tumor cells but also non-infected tumor cells. Heterologous DNA that is incorporated according to this specific aspect of the invention can encode proteins such as interleukins, preferably IL-2, IL-3 and/or IL-6. Other immune system stimulatory genes such as GM-CSF are incorporated in the adenoviral genome. Typically a cell that is transduced starts producing the encoded proteins that subsequently stimulate cells from the immune system such as natural killer cells (NK), T-cells, and macrophages to remove transduced and non-transduced tumor cells.

In another embodiment of the present invention an adenoviral replicon of the invention comprises heterologous DNA involved in induction of apoptosis to not only lyse the cells by the fact that the adenoviral replicon or adenovirus is replicating, packaged and released, but also to stimulate surrounding cells to exhibit detrimental effects such as apoptosis. In this specific part of the invention adenoviral replicon or adenovirus renders two separate killing actions: One being the lysis of the infected cells due to the replication, production and packaging of the virus particles; another being through the by-stander effect of the death inducing proteins encoded by the heterologous DNA such as the VP3 protein from chicken anemia virus, the cytosine deaminase protein, the nitroreductase protein, the thymidine kinase protein from herpes simplex virus, the linamarase protein (Pietersen et al 2000; Stackhouse et al 2000; Koyama et al 2000; Weedon et al 2000; Cortes et al 1998) or a functional equivalent thereof on surrounding cells.

In another embodiment of the invention replicating adenovirus comprises heterologous DNA encoding one or more proteins or functional equivalents thereof that have anti-angiogenic effects such as ATF-BPTI and some VEGF antagonists (Inoue et al 2000; Gagnon et al 2000). As a result of this, expressed protein(s) will inhibit further growth of blood vessels into tumor and thereby prevent further outgrowth of tumor cells and lower the possibility of a tumor to metastasize. Heterologous DNA can be incorporated in regions that are not involved in the replication machinery of the virus. Heterologous DNA can also be present in combinations with alterations in the viral genome that enhance the replication capability of said virus in tumor cells.

### Examples

### Example 1:Adenovirus entry and replication on human tumor cell lines

To compare the replication efficiency of different adenovirus serotypes it was investigated whether tumor cells support the entry of a particular adenovirus. Hereto, a small panel of human cancer cell lines was transduced with Ad5-based vectors carrying the fiber molecule derived from alternative serotypes. Human cancer cell lines tested for the susceptibility of different fiber chimaeric vectors were: A549 (Human lung epithelia, ATCC, CCL-185), Capan-1 (Human pancreas, ATCC, HTB-79), MCF-7 (Human breast, ATCC, HTB-22), BxPC3 (Human pancreas, ATCC, CRL-1687), U87 (Human astroglioma ECACC ref no. 89081402), SK-N-MC (Human neuroblastoma, ATCC, HTB-110), MIA-PaCa-2 (Human pancreas, ATCC, CRL-1420), Hs766T (Human pancreas, ATCC, HTB-134), HeLa (Human Cervix, ATCC, CCL-2), HepG2 (Human liver ECACC ref no. 85011430), SK-Cha-1 and Mz-Cha-1 (Both human cholangio carcinoma, a kind gift from Dr. Knuth, Frankfurt, Germany), OE19 and OE33 (Both human oesophagus ECACC ref no. 96071721 and 96070808 respectively), and TE-1, TE-2 and T-tn (All three human squamous cell carcinoma, A kind gift from Dr. Heideman, Amsterdam, The Netherlands). Of each cell line, 10⁵ cells were seeded in wells of 24-well plates in Dulbecco's modified Eagles medium (DMEM) supplemented with 10% fetal calf serum (FCS). Twenty-four hours later, cells were exposed to a virus dose of 1000 virus particles per cell of each of the chimaeric vectors indicated in **Table A**. Forty-eight hours after virus addition cells were washed twice with 1 ml PBS after which cells were lysed by adding 100 ml of lysis buffer. Luciferase transgene activity was determined using a bioluminescence machine and the luciferase assay kit from Promega (catalog nr. E-1501) following the instructions provided by the manufacturers. The results shown in **Table A** reflect the ability of the different adenoviral vectors to enter the tumor cell. Also shown in **Table A** are results obtained after screening each cell type for the presence of the Coxsackie adenovirus receptor (CAR) which is the primary receptor for adenovirus serotype 5. The results of these experiments thus show that Ad5 and the chimaeric fiber viruses selected are able to enter all tumor cells tested albeit with different efficiencies.

### Example 2: Influence of virus entry

In a next experiment we tested the influence of virus entry on the ability of wild type adenoviruses to replicate on the panel of human cell lines. As a positive control for virus viability, PER.C6 cells were taken since all viruses could be generated on this human cell line. We generated Ad5 vectors carrying the fiber molecule of several other adenovirus serotypes, i.e. Ad13, Ad16, Ad35, and Ad51. Virus was added at a dose of 10 virus particles per cell on different cell lines and the presence and robustness of CPE was scored 6 and 10 days after virus administration. The results of these experiments are shown in **Table B**. To interpret these results and discriminate between virus entry and replication the scheme as depicted in **Table C** was developed.

The situations numbered 2, 3, 7, and possibly 5, 6, and 8 in **Table C** (depending on the onset and robustness of CPE) could result in vectors that are better suited to use as recombinant vectors for cancer cell line transduction and replication. Situation (2) on a certain cell line could mean that another fiber on Ad5 is an improvement over Ad5. Situation (3) on a certain cell type could mean that another serotype must be used instead of Ad5. Situation (5) on a certain cell type could mean that another serotype might be better compared to Ad5, depending on the time of onset and robustness of CPE. Situation (6) on a certain cell type could mean that another fiber on Ad5 might be better compared to Ad5, depending on the time of onset and robustness of CPE. Situation (7 and 8) on a certain cell type could mean that either another serotype or another fiber might be better compared to Ad5.

*Situation (1)* is non-conclusive since the lack of Cyto Pathological Effect (CPE) observed with both adenoviruses can be due to either entry or the ability to replicate on such a cell line. Examples of this situation are U87, MiaPaca-2, and SK-N-MC.

*Situation (2+6)* : either Ad5 or the fiber-modified Ad5 vector gives CPE. In situation (2) Ad5 can probably replicate but not enter whereas AdX can enter but not replicate. In situation (6) a fiber-modified vector is better as compared to Ad5 if a higher CPE score is found. Examples of these situations are found with all fiber -modified vectors on Hs776T, and for Ad5/Ad35/Ad5Fib35 on MCF-7.

*Situation (3+7)* : Either AdX or Ad5FibX give CPE. In situation (3) another serotype is required to obtain replication in a certain cancer cell line. This situation was not observed using this relatively small panel of human cell lines. In situation (7) Ad5 and AdX can both replicate, but entry of Ad5 is impaired. This situation was not observed using this relatively small panel of human cell lines.

*Situation (4)* : only Ad5 gives CPE, thus states that AdX can at least not enter the cell since Ad5FibX cannot replicate. An example of this situation is found for Ad5/ Ad13/ Ad5Fib13 on U251. Here, Ad5 is superior compared to the other vectors and fiber-modified viruses tested.

*Situation (5):* both Ad5 and AdX give CPE but not Ad5FibX is unlikely to exist and is also not observed.

*Situation (8):* similar to situation 7 however Ad5 itself is able to replicate. A better vector compared to Ad5 depends on the time of onset and robustness of CPE. Examples of this situation are found for Ad5/Ad35/Ad5Fib35 on Capan-1 and BxPC3, or for Ad5/Ad16/Ad5Fib16 on Capan-1.

The results using this panel of human cancer cell lines show that either another adenovirus serotype or exchange of the fiber molecule of Ad5 for a fiber molecule from another serotype can result in a vector which has a better entry and/or replication advantage. Interestingly, on several human cancer cell lines we observed that all B-group derived vectors tested (16, 35, 51) did as good as or better than Ad5. This experiment was repeated, thereby taking all subgroup B adenovirus wild types (except 51). In this experiment CPE was determined at 3 days and 6 days after virus exposure (10 virus particles per cell). The results of this experiment are shown in **Table D.** These results show that except for U251, B-group serotypes replicate better compared to Ad5 on all human cancer cell lines tested which represent human pancreas, breast, and lung tumor cell lines.

### Example 3: Replication of subgroup D viruses

In a next experiment subgroup D adenoviruses were compared with Ad5 for their ability to replicate on human tumor cells. The experiment was performed as described above (10 virus particles of each virus per cell) and CPE was scored on day 3 and day 6. The results of these experiments are shown in **Table E**. In contrast to the positive control PER.C6 and as compared to A549, many of the D-group adenoviruses (21 out of 33 tested = 67%) replicate very poorly on the selection of human tumor cells. It is important to note that all cell lines were infected simultaneously with the same adenoviral batches, indicating that small differences in the quality of virus batches due to different treatments such as purifications or storage conditions cannot account for the observed differences in onset or robustness of CPE.

### Example 4: Generation of progeny virus

For the spread of virus through a tumor mass it is important that progeny virus is formed. When scoring CPE one scores for the toxicity of the virus infection and not for progeny formation although clear CPE usually indicates a lytic virus infection accompanied with release of progeny. To investigate whether virus is indeed generated with the formation of CPE we harvested the cells when CPE had occurred of some cell lines and of some viruses. Cells were frozen and centrifuged for 5 minutes at 1750 rpm. Of the supernatant 250 ml was added to 2750 DMEM/10% FCS. This 3 ml was used to infect a new layer of tumor cells and the formation of CPE scored. The results of this experiment are shown in **Table F**. These results show that life virus progeny is generated.

### References

Aarnoudse CA, van der Poul PB, Heemskerk B, Schrier PI (1999). Interleukin-2 induced, melanoma specific T cells recognize CAMEL, an unexpected translation product of LAGE-1. Int, J. Cancer 82(3), p442-448.

Cortes ML, De Felipe P, Martin V, Hughes MA, Izquierdo M (1998). Succesful use of a plant gene in the treatmentt of cancer in vivo. Gene Ther. 5(11), p1499-1507.

D'Ambrosio E, Del Grosso N, Chicca A, Midulla M (1982). Neutrlizing antibodies against 33 human adenoviruses in normal children in Rome. J. Hyg. Camb. 89, pp155-161.

De Jong JC, Wermenbol, AG, Verweij-uijterwaal MW, Slaterus KW, Werthheim-van Dillen P, van Doornum GJJ, Khoo SH, Hierholzer JC (1999). Adenoviruses from human immunodeficiency virus-infected individuals, including two strains that represent new candidate serotypes Ad50 and Ad51 of species B1 and D respectively. J. Clin. Microbiol. 37(12), pp3940-3945.

Francki, R.I.B., Fauquet, C.M., Knudson, D.L. and Brown, F. (1991) Classification and nomenclature of viruses. Fifth report of the international Committee on taxonomy of viruses. Arch. Virol. Suppl. 2: 140-144.

Gagnon ML, Bielenberg DR, Gechtman Z, Miao HQ, Takashima S, Soker S, Klagsbrun M (2000). Identification of a soluble neurophilin-1 that binds Vascular endothelial growth factor: in vivo expression and antitumor activity. Proc Natl Acad Sci USA 97(6), p2573-2578.

Hierholzer, J.C., Wigand, R., Anderson, L.J., Adrian, T., and Gold, J.W.M. (1988) Adenoviruses from patients with AIDS: a plethora of serotypes and a description of five new serotypes of subgenus D (types 43-47). J. Infect. Dis. 158, 804-813.

Hierholzer, J.C. (1992) Adenovirus in the immunocompromised host. Clin. Microbiol Rev. 5, 262-274.

Inoue T, Kibata K, Suzuki M, Nakamura S, Motoda R, Orita K (2000). Identification of a vascular endothelial growth (VEGF) antagonist, sFlt-1, fro ma human hemopoietic cell line NALM-16. FEBS lett 469(1), p14-18.

Jager E, Maeurer M, Hohn H, Karbach J, Jager D, Zidianakis Z, Bakhshandeh-Bath A, Orth J, Neukirch C, Necker A, Reichert TE, Knuth A (2000). Clonal expansion iofMelan-A-specific cytotoxic T lymphocytes in a melanoma patient responding to continued immunization with melanoma-associated peptides. Int. J. Cancer 86(4), p538-547.

Kawakami Y (2000). New cancer therapy by immunomanipulation: development of immunotherapy for human melanoma as a model system. Cornea 19(3 suppl.),p2-6.

Khoo, S.H., Bailey, A.S., De Jong, J.C., and Mandal, B.K. (1995). Adenovirus infections in human immunodeficiency virus-positive patients: Clinical features and molecular epidemiology. J. Infect. Dis 172, 629-637

Koyama F, Sawada H, Fujii H, Hirao T, Ueno M, Hamada H, Nakano H (2000). Enzyme/prodrug gene therapy for human colon cancer cells using adenovirus -mediated transfer of the escherichia coli cytosine deaminase gene driven by a CAG promoter associated with 5-fluorocytosine administration. J. Exp. Clin. Cancer Res. 19 (1), p75-870.

Mackenzen A, Herbst B, Chen JL, Kohler G, Noppen C, Herr W, Spagnoli GC, Cerundolo V, Lindemann A (2000). Phase I study in melanoma patients of a vaccine with peptide-pulsed dendritic cells generated in vitro from CD34(+) hemopoietic progenitor cells. Int. J. Cancer 86(3), p385-392.

Pietersen A, Noteborn HM (2000). Apoptin Adv. Med. Biol. 465, p 153-161.

Schnurr, D and Dondero, M.E. (1993) Two new candidate adenovirus serotypes. Intervirol. 36, 79-83.

Stackhouse MA, Pederson LC, Grizlle WE, Curiel DT, Gebert J, Haack K, Vickers SM, Mayo MS, Buchsbaum DJ (2000). Fractionated radiation therapy in combination with adenoviral delivery of the cytosine deaminase gene and 5-fluorocytosine enhances cytotoxic and anti tumor effects in human colorectal and cholangiocarcinoma models. Gene Ther. 7(12), p1019-1026.

Tsukamoto K, Ueda M, Hirata S, Osada A, Kitamura R, Takahashi T, Ichishasi M, Shimada S (2000). Gp100 mRNA is more sensitive than tyrosine mRNA for RT-PCR amplification to detect circulating melanoma cells in peripheral blood of melanoma patients. J, Dermatol Sci 23(2), p126-131.

Tuting T, Steitz J, Bruck J, Gambotto A, Steinbrink K, Deleo AB, Robbins P, Knop J, Enk AH (1999). Dendritic cell based genetic immunization in mice with a recombinant adenovirus encoding murine TRP2 induces effective anti-melanoma immunity. J. Gene Med. 1(6), p 400-406.

Weedon SJ, Green NK, McNeish IA, Gilligan MG, Mautner V, Wrighton CJ, Mountain A, Young LS, Kerr DJ, Searle, PF (2000). Sensitation of human carcinoma cells to the prodrug CB1954 by adenovirus vector-mediated expression of E.coli nitroreductase. Int. J. Cancer 86(6), p848-854.

Yang S, Kittlesen D, Slingluff CL, Vervaert CE, Seigler HF, Darrow TL (2000). Dendritic cells infected with a vaccinia vactor carrying the human gp100 gene simutaneously present multiple specificities and elicit high affinity T cells reactive to multiple epitopes and restricted by HLA-A2 and - A3. J. Immunol 164(8), p4204-4211.

**Table A**

| Cell line | CAR* | Ad5** | Fib11 | Fib13 | Fib16 | Fib32 | Fib35 | Fib51 |
|---|---|---|---|---|---|---|---|---|
| A549 | ++ | 10⁶ | 10⁶ | 10⁶ | 10⁶ | 10⁶ | 10⁶ | 10⁶ |
| Capan-1 | ++ | 10⁵ | 10⁵ | 10⁶ | 10⁶ | 10⁵ | 10⁶ | 10⁶ |
| MCF-7 | ND | 10⁶ | 10⁶ | ND | 10⁷ | 10⁷ | 10⁶ | 10⁶ |
| BxPC3 | ++ | 10⁶ | 10⁵ | 10⁵ | 10⁵ | 10⁴ | 10⁵ | 10⁵ |
| U-87 | -/+ | 10⁵ | 10³ | ND | 10³ | 10⁴ | 10³ | 10³ |
| SK-N-MC | -/+ | 10⁶ | 10⁶ | ND | 10⁵ | 10⁴ | 10⁴ | 10⁵ |
| MIA-PaCa-2 | ++ | 10⁷ | 10⁷ | 10⁷ | 10⁷ | 10⁶ | 10⁷ | 10⁷ |
| Hs 766-T | ++ | 10⁶ | 10⁶ | 10⁶ | 10⁶ | 10⁴ | 10⁶ | 10⁶ |
| Hela | ++ | 10⁶ | ND | ND | 10⁴ | 10³ | ND | 10⁴ |
| HEP-G2 | ++ | 10⁷ | ND | ND | 10⁶ | 10⁵ | ND | 10⁵ |
| Sk-Cha-1 | ++ | 10⁵ | 10⁵ | 10⁵ | 10⁶ | 10⁴ | 10⁵ | 10⁴ |
| Mz-Cha-1 | ++ | 10⁵ | 10⁵ | 10⁵ | 10⁶ | 10⁴ | 10⁶ | 10⁶ |
| OE-19 | ++ | 10⁴ | 10⁴ | 10⁴ | 10⁵ | 10³ | 10⁴ | 10⁵ |
| OE-33 | ++ | 10⁶ | 10⁶ | 10⁶ | 10⁶ | 10⁵ | 10⁶ | 10⁷ |
| TE-1 | ++ | 10⁵ | 10⁶ | 10⁶ | 10⁶ | 10³ | 10⁶ | 10⁶ |
| TE-2 | ++ | 10⁵ | 10⁵ | 10⁵ | 10⁵ | 10³ | 10⁵ | 10⁵ |
| T-tn | ++ | 10⁵ | 10³ | 10³ | 10⁴ | 10² | 10³ | 10⁴ |

**Table B**

| Multiplicity of infection used: 10 virus particles per cell | | | | | | | | |
|---|---|---|---|---|---|---|---|---|
| Cell line | Day | Ad5 | Ad13 | Ad5Fib13 | Ad16 | Ad5Fib16 | Ad35 | Ad5Fib35 |
| U251 | 6 | +++ | - | - | + | + | + | ++ |
| U251 | 10 | ++++ | - | ++ | + | + | + | ++++ |
| HS776T | 6 | - | - | + | - | + | - | ++ |
| HS776T | 10 | ++ | - | ++++ | + | ++++ | + | ++++ |
| U87 | 6 | - | - | - | - | - | - | - |
| U87 | 10 | - | - | - | - | - | - | - |
| Capan-1 | 6 | + | - | - | ++ | +++ | ++ | +++ |
| Capan-1 | 10 | ++++ | ++++ | ++++ | ++++ | ++++ | ++++ | ++++ |
| MCF-7 | 6 | +++ | - | +++ | + | ++ | - | ++++ |
| MCF-7 | 10 | ++++ | + | ++++ | ++ | ++++ | ++++ | ++++ |
| MiaPaca | 6 | - | - | - | - | - | - | - |
| MiaPaca | 10 | - | - | - | - | - | - | - |
| BxPC3 | 6 | ++ | - | + | ++ | ++ | +++ | +++ |
| BxPC3 | 10 | ++++ | - | ++ | +++ | +++ | ++++ | ++++ |
| SK-N-MC | 6 | - | - | - | - | - | - | - |
| SK-N-MC | 10 | - | - | - | - | - | - | - |
| A549 | 6 | ++++ | +++ | ++++ | ++++ | ++++ | ++++ | ++++ |
| A549 | 10 | ++++ | ++++ | ++++ | ++++ | ++++ | ++++ | ++++ |
| PER.C6 | 6 | ++++ | ++++ | ++++ | +++ | ++++ | ++++ | ++++ |
| PER.C6 | 10 | ++++ | ++++ | ++++ | ++++ | ++++ | ++++ | ++++ |
| Scoring - 0% CPE + 25% CPE ++ 50% CPE +++ 75% CPE ++++ 100% CPE | | | | | | | | |

**Table C**

| CPE observed with | | | replication | | entry | | situation |
|---|---|---|---|---|---|---|---|
| Ad5 | AdX | Ad5FibX | Ad5 | AdX | Ad5 | Adx | |
| - | - | - | ? | ? | ? | ? | (1) |
| - | - | + | Y | N | N | Y | (2) |
| - | + | - | N | Y | ? | Y | (3) |
| + | - | - | Y | ? | Y | N | (4) |
| + | + | - | Y | Y | Y | Y | (5) |
| + | - | + | Y | N | Y | Y | (6) |
| - | + | + | Y | Y | N | Y | (7) |
| + | + | + | Y | Y | Y | Y | (8) |
| AdX is a wild type adenovirus serotype other than adenovirus serotype 5 (Ad5). Ad5FibX is an Ad5 wild type vector carrying the fiber of a wild type adenoviruses other than Ad5. Scoring: - (no CPE), + (CPE), Y = Yes, N = No | | | | | | | |

**Table D**

| Cell line | Day | **Ad5** | Ad3 | Ad7 | Ad11 | Ad14 | Ad16 | Ad21 | Ad34 | Ad35 |
|---|---|---|---|---|---|---|---|---|---|---|
| U251 | 3 | + | - | - | - | - | - | - | - | - |
| U251 | 6 | +++ | - | - | + | - | - | - | - | - |
| Hs776T | 3 | - | ND | ND | - | ND | - | ND | ND | - |
| Hs776T | 6 | - | ND | ND | ++ | ND | + | ND | ND | + |
| Capan-1 | 3 | - | - | - | - | - | - | - | - | - |
| Capan-1 | 6 | + | - | - | ++++ | - | ++ | + | ++ | ++ |
| MCF-7 | 3 | - | - | - | + | - | + | - | - | - |
| MCF-7 | 6 | ++ | - | - | ++++ | - | ++ | + | ++ | - |
| BxPC3 | 3 | - | - | - | + | - | - | - | + | - |
| BxPC3 | 6 | ++ | - | - | ++++ | - | + | + | ++ | - |
| A549 | 3 | + | ++ | + | +++ | - | ++ | ++ | ++ | ++ |
| A549 | 6 | ++++ | ++++ | ++++ | ++++ | ++ | ++++ | ++++ | ++++ | ++++ |
| PER.C6 | 3 | +++ | + | +++ | ND | - | ++ | + | ++++ | ++ |
| PER.C6 | 6 | ++++ | ++ | ++++ | ++++ | + | +++ | +++ | ++++ | ++++ |
| Multiplicity of infection used: 10 virus particles per cell | | | | | | | | | | |

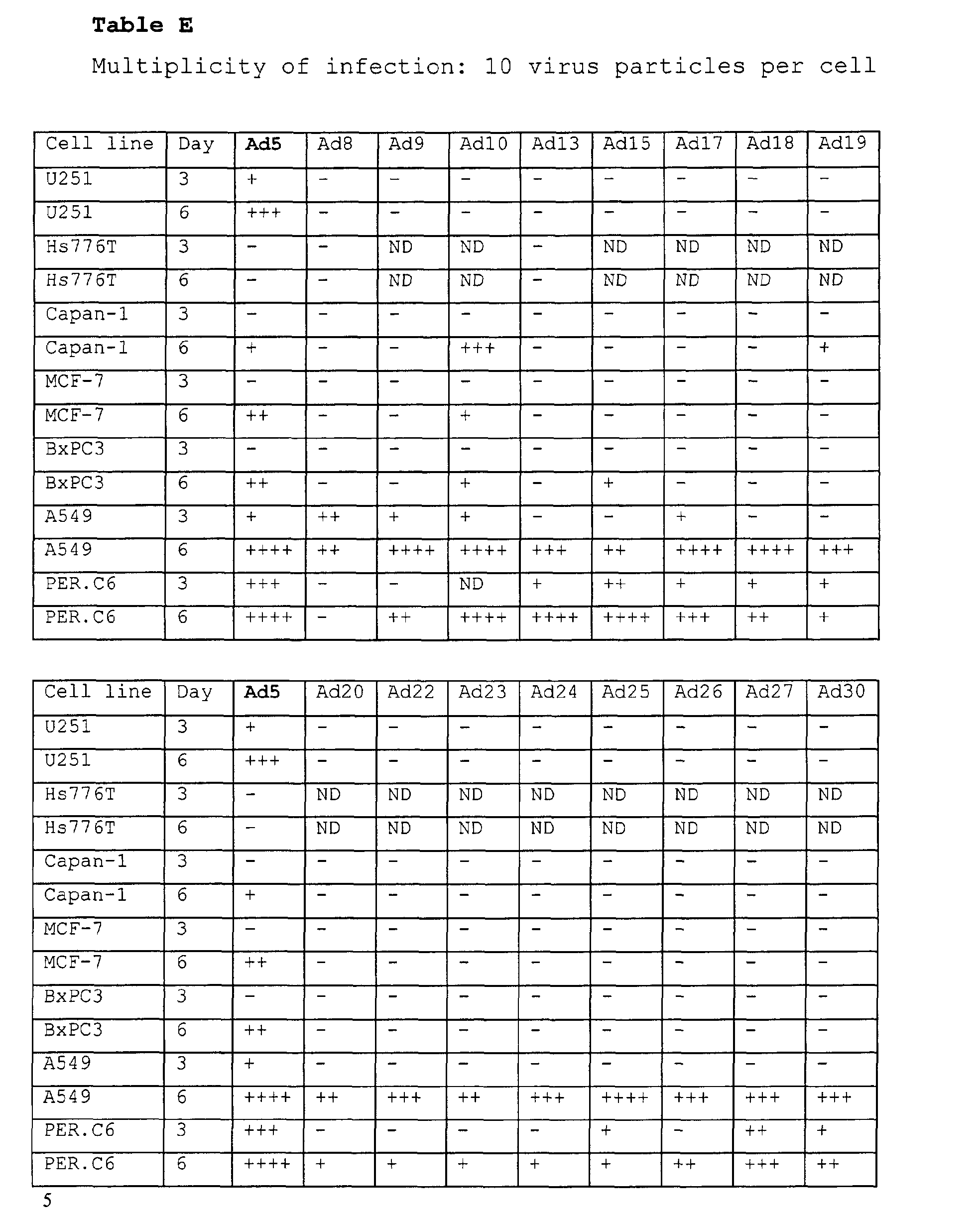

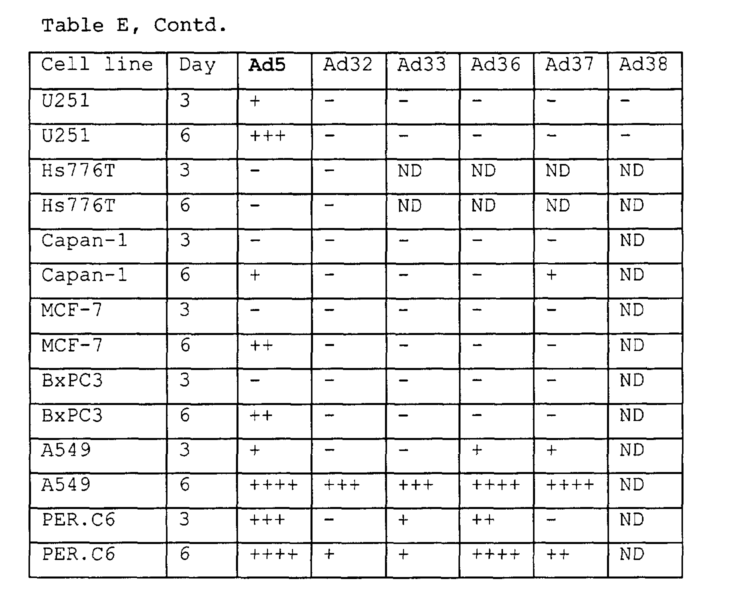

**Table F**

| Cell line | Ad5 | Ad5 | Ad11 | Ad11 | Ad16 | Ad16 | Ad51 | Ad51 |
|---|---|---|---|---|---|---|---|---|
| | 1^{st} | 2^{nd} | 1^{st} | 2^{nd} | 1^{st} | 2^{nd} | 1^{st} | 2^{nd} |
| A549 | ++++ | ++++ | ++++ | ND | ++++ | ++++ | ++++ | ++++ |
| BxPC3 | ++++ | +++ | ++++ | ++++ | ++++ | ++++ | ++++ | ND |
| Capan-1 | ++++ | ++ | ++++ | ND | ++++ | ND | ++++ | + |
| Hs766T | ++++ | ++ | ++++ | ++++ | ++++ | ND | ++++ | ND |
| PER.C6 | ++++ | ++++ | ++++ | ++++ | ++++ | ++++ | ++++ | ++++ |

## Claims

1. A method for identifying an adenoviral replicon capable of eliminating a target cell, comprising contacting a representative cell with said adenoviral replicon and observing any detrimental effect.

2. A method according to claim 1, wherein said adenoviral replicon comprises an adenovirus.

3. A method according to claim 2, wherein said adenovirus comprises a recombinant adenovirus.

4. A method according to any of claims 1-3, wherein said adenoviral replicon comprises DNA from at least one adenovirus serotype from subgroup B or C.

5. A method according to any of claims 1-4, wherein said adenoviral replicon comprises a chimaeric virus.

6. A method according to claim 5, wherein said chimaeric virus comprises a fusion between DNA from adenovirus serotype 5 and subgroup B adenoviral DNA.

7. A method according to any of claims 1-6, wherein said adenoviral replicon comprises an alteration in its E1 region and/or its E3 region.

8. A method according to any of claims 1-7, wherein said adenoviral replicon comprises a gene encoding a functional E1B 55K protein.

9. A method according to any of claims 1-8, wherein said adenoviral replicon comprises a change in the E1B gene.

10. A method according to any of claims 1-7 or 9, wherein said adenoviral replicon comprises an alteration resulting in the loss of E1B 55K function.

11. A method according to any of claims 1-10, wherein said adenoviral replicon comprises an exchange of a structural protein encoding nucleic acid or a part thereof for a structural protein encoding nucleic acid or a part thereof from another source.

12. A method according to claim 11, wherein said exchange comprises the replacement of a structural protein encoding nucleic acid or a part thereof from adenovirus serotype 5 for a structural protein encoding nucleic acid or a part thereof from subgroup C.

13. A method according to claim 11 or 12, wherein said structural protein encoding nucleic acid encodes a fiber protein.

14. A method according to any of claims 1-13, wherein said adenoviral replicon comprises a change of a gene encoding a protein involved in replication.

15. A method according to any of claims 1-14, wherein said adenoviral replicon comprises heterologous DNA.

16. A method according to claim 15, wherein said heterologous DNA comprises a nucleic acid encoding an antigen.

17. A method according to claim 16, wherein said antigen comprises a tumor-specific antigen.

18. A method according to claim 17, wherein said tumor-specific antigen comprises gp100 and/or CAMEL and/or FRAME and/or Mart-1 and/or Mage-1 and/or Melan-A and/or tyrosinase or a functional equivalent thereof.

19. A method according to claim 15, wherein said heterologous DNA comprises a nucleic acid encoding a detrimental effect inducing protein.

20. A method according to claim 19, wherein said detrimental effect inducing protein comprises the VP3 protein from chicken anemia virus and/or the cytosine deaminase protein and/or the nitroreductase protein and/or the thymidine kinase protein from herpes simplex virus and/or the linamarase protein or a functional equivalent thereof.

21. A method according to claim 15, wherein said heterologous DNA comprises a nucleic acid encoding a protein with anti-angiogenesis effects.

22. A method according to claim 21, wherein said protein comprises ATF-BPTI, a VEGF antagonist or a functional equivalent thereof.

23. A method according to any of claims 1-22, wherein said target cell comprises a neoplastic cell.

24. A method according to any of claims 1-23, wherein said target cell does not express a functional tumor suppressor gene product.

25. A method according to any of claims 1-24, wherein said target cell does not express a functional p53 protein and said adenoviral replicon comprises a change in the E1B gene.

26. A method according to any of claims 1-25, wherein said representative cell comprises a human cell.

27. A method according to any of claims 1-26, wherein said representative cell comprises a tumor cell.

28. A method according to claim 27, wherein said tumor cell is derived from a brain tumor, a gastro-intestinal tumor, a pancreatic tumor, a liver tumor, a mammary tumor, a cervix tumor, a lung tumor or a skin tumor or derivative thereof.

29. A method according to any of claims 1-28, wherein said detrimental effect comprises lysis.

30. An adenoviral replicon obtainable by a method according to any of claims 1-29.

31. A method for producing an adenoviral replicon according to claim 30 comprising contacting a producer cell with said adenoviral replicon and obtaining produced adenoviral replicon from said producer cell and/or culture medium.

32. A method according to claim 31, wherein said producer cell comprises a representative cell or derivative thereof.

33. A method according to claim 31 or 32, wherein said producer cell comprises a functional E1 region derived from adenovirus.

34. A method according to any of claims 31-33, wherein said producer cell comprises PER.C6 or a derivative thereof.

35. A method according to claim 31 or 32, wherein said producer cell comprises A549 or U87 or a derivative thereof.

36. A method for purifying an adenoviral replicon obtainable according to any of claims 1-35.

37. A pharmaceutical composition comprising an adenoviral replicon according to claim 30.

38. A method for treating a neoplastic condition, comprising administering a composition comprising an adenoviral replicon capable of eliminating a neoplastic cell to an individual having a neoplasm.

39. A method according to claim 38, wherein said neoplastic cell is a tumor cell.
